Europäisches Patentamt

**European Patent Office**

Office européen des brevets

(11) Publication number: **0 114 037**
A2

# (12) EUROPEAN PATENT APPLICATION

(21) Application number: **83830268.5**

(51) Int. Cl.³: **A 61 B 3/02**

(22) Date of filing: **16.12.83**

(30) Priority: **17.01.83 IT 1914283**

(71) Applicant: **Carlevaro, Gianfranco, Via Spiga, 7, I-20121 Milano (IT)**

(43) Date of publication of application: **25.07.84 Bulletin 84/30**

(72) Inventor: **Carlevaro, Gianfranco, Via Spiga, 7, I-20121 Milano (IT)**

(84) Designated Contracting States: **AT CH DE FR GB LI NL**

(74) Representative: **Cicogna, Franco, Ufficio Internazionale Brevetti Dott.Prof. Franco Cicogna Via Visconti di Modrone, 14/A, I-20122 Milano (IT)**

(54) **Ophthalmoscopic instrument for testing in a quick and recordable way the main clinical parameters of sight.**

(57) The instrument comprises two like elements of substantially shovel shape and different colours, provided with a central small hole (2) and a larger hole (3) offset from one another and including a respective disk-shaped portion (1) thereon there are defined alphabetic marks and, on the opposite side, four segments (5), arranged in a cross arrangement. The instrument further comprises a handle portion, thereon there are reproduced the alphabetical marks or signs (4) which are symmetrically arranged with respect to the central small hole (2) and may be replaced by a plurality of differentiated operatively equivalent graphic marks or signs.

The present invention relates to an ophthalmoscopic instrument for testing in a quick and recordable way the main clinical parameters of sight.

As it is known, by examining the bottom of the eye it is possible to study the conditions of the retina and retina vessels, as well as to detect possible alterations and diseases of the eye itself.

Also known is the fact that an oculist may greatly aid the diagnostic work of a physician or neurologist in the case of several diseases originally of extraocular type which affect, with a more or less rate and characteristic symptoms, the eyes of the patient.

In order to carry out a valid and useful oculistic test it is not possible to merely examine the bottom of the eye and sight sharpness. Infact a normal sight sharpness and normal eye bottom do not exclude alterations of the eye or brain, which alterations may be detected, on the contrary, by testing two other clinical parameters, that is the field and diplopia.

The presently available instruments for detecting the two above mentioned clinical parameters are however very complex and difficult to use, thereby they are scarcely adopted.

That lack inevitably introduces serious delays in tests which are very important in order to detect possible bad deseases.

0114037

-3-

Accordingly, the task of the present invention is to overcome the hereinabove mentioned drawbacks, by providing such an ophthalmoscopic instrument which is very simple construction-wise and very easy to be used.

Within that task, a main object of the present invention is to provide such an ophthalmoscopic instrument which affords the possibility of extending to generic medicine the essential sight subjective tests which presently may be carried out only by a specialist.

Another object of the present invention is to provide such an ophthalmoscopic instrument which affords the possibility of carrying out a general test of sight, with good results.

Yet another object of the present invention is to provide such an ophthalmoscopic instrument which is very practical and of reduced cost, thereby affording the possibility of application in the preventive medicine field.

According to one aspect of the present invention, the above task and objects, as well as yet other objects which will become more apparent hereinafter, are achieved by an ophthalmoscopic instrument, characterized in that it comprises two like elements of substantially shovel shape and different colours, provided with a central small hole and a larger hole offset from one another, on one of the sides of the disk shaped portion of said elements there being defined alphabetic marks and on the opposite
side of said disk-shaped portion there being formed,
starting from said small hole, four segments,
arranged in a cross-arrangement, said instrument
further comprising a handle portion thereon said
alphabetical marks or signs are reproduced.

In particular, the above mentioned offset
large hole may advantageously be used by the
physician for tracing, on a card, circular shapes by
following the periphery of said hole.

Within those circles the physician will
record, for each eye, what the patient perceives by
viewing either surfaces of the shovel.

Further characteristics and advantages of
the ophthalmoscopic instrument according to the
present invention will become more apparent from the
following detailed description of a preferred
embodiment of said instrument, being illustrated, by
way of an example and not of limitation, in the
figures of the accompanying drawings, where:

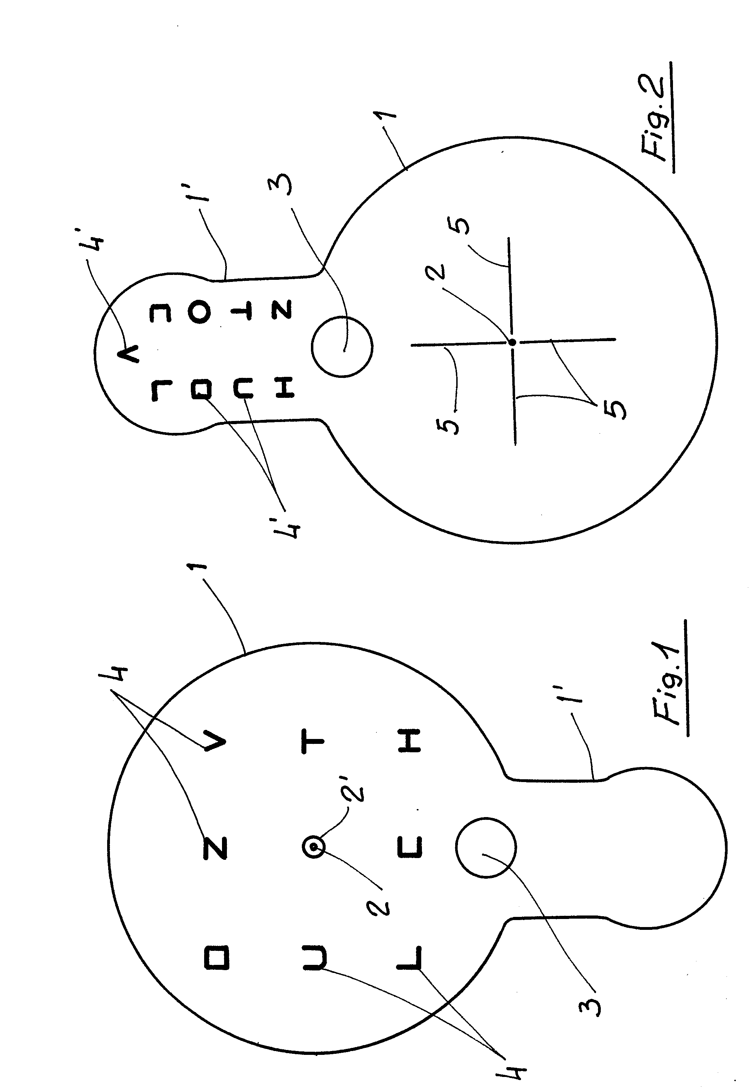

Fig.1 illustrates one of the sides or faces
of one of the two like elements forming the instrument
according to the present invention;

fig.2 illustrates the opposite side of the
same element, as seen in a reversed position;

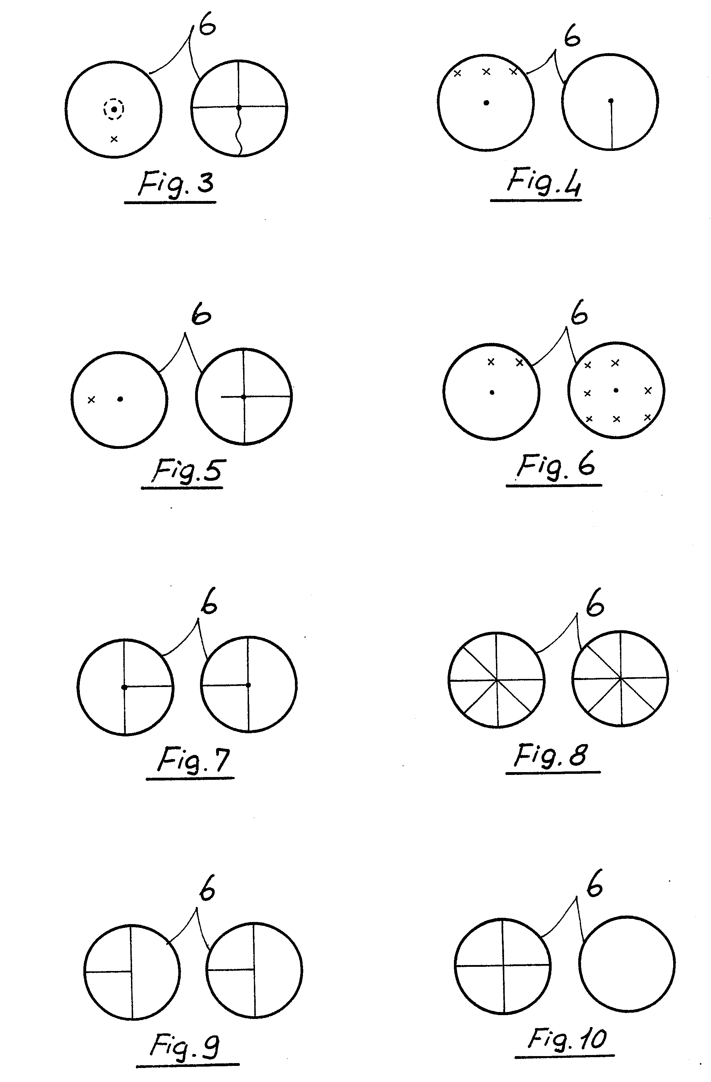

figs.3 to 10 illustrate some possible
examples relating to the perceiving of cross segments
and alphabetic signs or marks, for the left and
right eyes of patients subjected to the test; in
particular fig.8 relating to the case therein the shovel is observed also after a rotation thereof
through 45° in its plane;

figs.5 and 6 illustrate some examples of
the perceiving of alphabetic signs(in particular
the small crosses traced by the physician are
indicative of the not perceived signs or marks);
and

figs.7,8,9 illustrate some examples of a
faulty perceiving of the cross segments respectively
in the case of a hypophysis tumour and cerebral
thrombosis(the not perceived segments are not
signed):these examples are pathognomic..

With reference to the figures of the
accompanying drawings,the ophthalmoscopic instrument
according to the present invention comprises two
like elements both consisting of a disk-shaped
portion 1,provided with a radially extending lug
1' and of substantially shovel shape.

The mentioned two like elements are
preferably differently coloured,for example in red
and yellow,and are each provided with a central
small hole 2 and an offset greater hole indicated
at 3.

On one side thereof,the mentioned shovel
elements are provided with a plurality of alphabetic
marks or signs 4,or any other suitable differentiated
graphic signs,which are symmetrically arranged with
respect to the central small hole which is encompassed by a small circle,2'.

On the opposite side,the like elements are provided with four segments 5,arranged cross-wise,
radially of the mentioned central small hole and,at
the lug 1',they are provided with a reproduction of
the mentioned alphabetic or graphic signs or marks.

More specifically,the cross segments and the
alphabetic or graphic signs or marks,as formed on the
disk-shaped portions of the shovel elements,are of a
weak lightness contrast and accordingly they may be
perceived with greater difficulty,in order to better
detect sight alterations.

The central small hole 2,on the other hand,
is effective to be used as a viewing point and,
jointly to the cross segments and mentioned alphabetic
or graphic signs,as a perception rest.

It should be noted that the sizes of the disk
shaped portions of the shovel elements are such that
they are effective to afford the possibility of
carrying out,in a field test at a distance of about
40 cm,the scanning of an area of 10 sr about the
viewing point.

The large hole 3,in turn,is effective for
detecting diplopia and properly recording the viewing
field.

It should also be noted that the above mentioned
shovel elements are so shaped that they may be
advantageously used for alternatively screening either
eye of a patient being tested.

In actual practice,by causing the patient to
observe by a single free eye,either one or the other
side or face of the shovel,as held at a suitable
distance,the physician may record,inside the mentioned

circles 6,what the patient has perceived with the possibility of evaluating,according to a predetermined procedure,both possible sight defects and possible retina alterations,as well as possible brain alterations.

Moreover it will be possible to detect a diplopia,by causing the patient to observe with his two eyes the large hole of the shovel element at a distance of 50 cm.In order to better classify the diplopia type,the examiner will displace the shovel element in eight directions,that is,in the order: upwardly,downwardly,upwardly to the right,upwardly to the left,downwardly to the right and downwardly to the left.

From the above disclosure and the figures of the accompanying drawings,the great functionality and use facility characterizing the ophthamoscopic instrument according to the present invention will be self evident.

While the subject instrument has been hereinabove disclosed and illustrated with reference to a preferred embodiment thereof,it should be noted that it is susceptible to many modifications and variations, all whereof do not depart from the spirit and scope of the invention,as defined in the accompanying claims.

-8-

CLAIMS

1- An ophthalmoscopic instrument, characterized in that it comprises two like elements of substantially shovel shape and different colours, provided with a central small hole (2) and a larger hole (3) offset from one another, on one of the sides of the disk shaped portion (1) of said elements there being defined alphabetic marks (4) and on the opposite side of said disk shaped portion (1) there being formed, starting from said small hole (2), four segments (5), arranged in a cross arrangement, said instrument further comprising a handle portion (1') thereon said alphabetical marks or signs are reproduced.

2- An ophthalmoscopic instrument according to the preceding claim, characterized in that said two elements comprise a respective disk-shaped portion (1), provided with a radially extending lug (1'), and being respectively coloured in red and yellow.

3- An ophthalmoscopic instrument according to claim 1, characterized in that said alphabetical marks or signs (4) are symmetrically arranged with respect to said central small hole (2) and may be replaced by a plurality of differentiated operatively equivalent graphic marks or signs.

4- An ophthalmoscopic instrument according to claim 1, characterized in that said cross segments (5) and alphabetic marks, or graphic marks, (4), formed on said disk-shaped portions (1) of said shovel elements
have a weak lightness contrast relationship with
respect to the background colour of said disk-shaped
portions.

5- An ophthalmoscopic instrument according to claim
1,characterized in that the disk-shaped portions
(1) of said shovel elements are so sized as to
afford the possibility of carrying out,during a
field test,at a distance of about 40 cm,a scanning
of an area of 10 sr about the viewing point.

6- An ophthalmoscopic instrument according to
claim 1,characterized in that said shovel elements
are so shaped that they may be used for screening
alternatively either eye of the patient to be subjected to the test.

7- An ophthalmoscopic instrument according to the
preceding claims and substantially as disclosed and
illustrated for the intended objects.

Fig.1

Fig.2

1/2

0114037

0114037

Fig. 3

Fig. 4

Fig. 5

Fig. 6

Fig. 7

Fig. 8

Fig. 9

Fig. 10